# EUROPÄISCHE PATENTSCHRIFT

(11) **EP 0 866 055 B1**
(45) Veröffentlichungstag und Bekanntmachung des Hinweises auf die Patenterteilung: **03.07.2002**
(21) Anmeldenummer: 98104136.1
(22) Anmeldetag: 09.03.1998
(51) Int. Cl.: C07C 211/36, C07C 209/68, C07C 209/62

(54) **Verfahren zur Herstellung von 1-Amino-1-methyl-3(4)-amino-methylcyclohexan**
Pocess for the preparation of 1-amino-1-methyl-3(4)-aminomethylcyclohexane
Procédé pour la préparation du 1-amino-1-méthyl-3(4)-aminométhylcyclohexane

(30) Priorität: 20.03.1997 DE 19711549
(43) Veröffentlichungstag der Anmeldung: 23.09.1998
(73) Patentinhaber: BAYER AG, 51368 Leverkusen (DE)
(72) Erfinder: Fischer, Konrad, Dr., 51519 Odenthal (DE); Wilmes, Oswald, Dr., 51061 Köln (DE); Arlt, Dieter, Prof. Dr., 32657 Lemgo (DE); Casser, Carl, Dr., 51061 Köln (DE); Maas, Peter, 6365 Puth (NL); Woestenborghs, Pierre, 3650 Dilsen (BE); Van der Knaap, Theo, Dr., 6120 Grevenbicht (NL); Reintjens, Raf, 6360 Schimmert (NL)

(56) Entgegenhaltungen:
- EP-A- 0 153 561
- EP-A- 0 193 828
- EP-A- 0 415 213
- DE-A- 4 401 929

## Beschreibung

Die Erfindung betrifft ein neues Verfahren zur Herstellung von 1-Amino-1-methyl-3(4)-aminomethylcyclohexan (AMCA), dem Vorprodukt zur Herstellung von 1-Isocyanato-1-methyl-3(4)-isocyanatomethylcyclohexan (IMCI).

In der DE-A 4 401 929 wird ein Verfahren zur Herstellung von 1-Amino-1-methyl-3(4)-aminomethylcyclohexan (AMCA) beschrieben, das durch das folgende Reaktionsschema beschrieben ist.

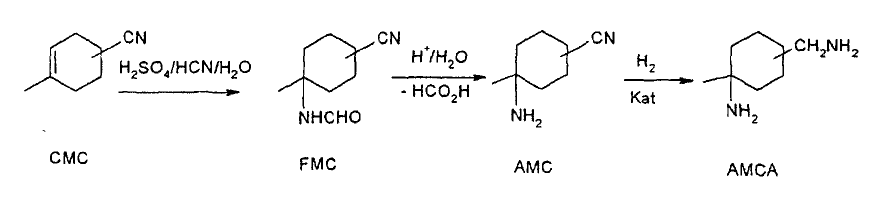

Nach diesem Vorschlag wird in einer Ritterreaktion das als Ausgangsprodukt dienende 4(5)-Cyano-1-methylcyclohexen (CMC) mit Blausäure in Gegenwart von Schwefelsäure zum 1-Formamido-1-methyl-3(4)-cyanocyclohexan (FMC) umgesetzt. In einem weiteren Reaktionsschritt wird anschließend das 1-Formamido-1-methyl-3(4)-cyanocyclohexan (FMC) sauer zum 1-Amino-1-methyl-3(4)-cyanocyclohexan (AMC) hydrolisiert, welches dann in an sich bekannter Weise zum 1-Amino-1-methyl-3(4)-aminomethylcyclohexan (AMCA) hydriert wird. Um bei der Ritterreaktion hohe Selektivitäten an FMC zu erhalten, ist ein erheblicher Blausäureüberschuß erforderlich, so daß dieses Verfahren nur mit erheblichem sicherheitstechnischem Aufwand betrieben werden kann.

In der Europäischen Patentanmeldung EP-A 0 153 561 wird ein Verfahren zur Herstellung von 1-Amino-1-methyl-3(4)-aminomethylcyclohexan (AMCA) beschrieben, das durch das folgende Reaktionsschema gekennzeichnet ist.

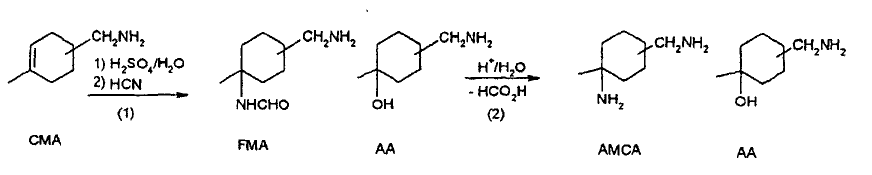

Bei diesem Verfahren wird die Ritterreaktion (1) in der Art und Weise durchgeführt, daß im ersten Schritt 4(5)-Aminomethyl-1-methylcyclohexen (CMA) in Schwefelsäure eingetragen wird und anschließend zu dieser Reaktionsmischung Blausäure bei 10° bis 50°C zudosiert wird. Bei dieser Verfahrensweise werden auch nach vier Stunden ca. 13 % an3(4)-Aminomethyl-l-methylcyclohexanol (AA) erhalten, welches destillativ nur schwer vom l-Amino-1-methyl-3(4)-arninomethylcyclohexan (AMCA) getrennt werden kann (siehe Beispiel 1a aus EP-A 0 153 561). Für die sich anschließenden Hydrolysereaktion (2) werden nach dem beschriebenen Verfahren Reaktionszeiten von drei Stunden benötigt. Aufgrund der für die Ritterreaktion (1) und Hydrolysereaktion (2) benötigten langen Reaktionszeiten ist dieses Verfahren mit erheblichen Investitions- und Energiekosten verbunden. Im Europäischen Patent EP-A 0 153 561 wird als Ausgangsmaterial für die Ritterreaktion (1) auch das 3(4)-Aminomethyl-1-methylcyclohexanol (AA) verwendet, wobei die hierbei erzielte Selektivität jedoch nur ca. 75 % beträgt. Somit ergibt sich nach dem EP-A 0 153 561 eine Gesamtselektität von ca. 81 %.

Es wurde nun ein Verfahren zur Herstellung von 1-Amino-1-methyl-3(4)-aminomethylcyclohexan (AMCA), aus 4(5)-Aminomethyl-1-methylcyclohexen (CMA) gefunden, bei dem in kurzen Reaktionszeiten hohe Selektivitäten erzielt werden und welches somit deutliche wirtschaftliche Vorteile gegenüber den bisher beschriebenen Verfahren aufweist.

Gegenstand der Erfindung ist ein Verfahren zur Herstellung von 1-Amino-1-methyl-3(4)-aminomethylcyclohexan dadurch gekennzeichnet, daß man
a) in der ersten Reaktionsstufe 4(5)-Aminomethyl-1-methylcyclohexen (CMA), Blausäure und wäßrige Schwefelsäure gleichzeitig bei Temperaturen von 60°C bis 120°C, bevorzugt bei 80°C bis 120°C umsetzt, wobei man pro Mol CMA 1-3 Mol Blansäure verwendet, und
b) in der zweiten Reaktionsstufe nach Wasserzugabe das in der ersten Reaktionsstufe gebildete 1-Formamido-1-methyl-3(4)-aminomethylcyclohexan (FMA) und nicht umgesetzte Blausäure hydrolisiert und
c) aus dem in der zweiten Reaktionsstufe erhaltenen Reaktionsgemisch 1-Amino-1-methyl-3(4)-aminomethylcyclohexan (AMCA) nach vorhergehender Basenzugabe und ggf. nach vorheriger Ameisensäureentfernung durch Extraktion isoliert.

Bei dem erfindungsgemäßen Verfahren werden in der ersten Reaktionsstufe pro Mol CMA ein bis drei Mol Blausäure, bevorzugt 1,1 bis 1,7 Mol Blausäure und zwei bis drei Mol Schwefelsäure, bevorzugt 2,2 bis 2,7 Mol Schwefelsäure, verwendet. Der Wassergehalt der Schwefelsäure wird so eingestellt, daß sich ein Molverhältnis von CMA zu Wasser von eins zu sieben bis eins zu zwei, bevorzugt von eins zu sechs bis eins zu drei ergibt.

Bei dem erfindungsgemäßen Verfahren betragen die für einen Umsatz größer 99 % benötigten Reaktionszeiten für die erste Reaktionsstufe drei bis 60 Minuten, wobei hohe Temperaturen sowie Blausäure- und Schwefelsäureüberschüsse die Reaktion beschleunigen.

Die erste Reaktionsstufe des erfindungsgemäßen Verfahrens kann sowohl diskontinuierlich in einem Rührkessel, als auch kontinuierlich ausgeführt werden, wobei die kontinuierliche Verfahrensweise bevorzugt ist. Die kontinuierliche Durchführung der ersten Reaktionsstufe kann z.B. in einer Rührkesselkaskade, einem Schlaufenreaktor oder in einem Rohrreaktor sowie in einer beliebigen Hintereinanderschaltung dieser Reaktortypen ausgeführt werden. Bevorzugt ist die Kombination eines Schlaufen- mit einem Rohrreaktor, da hierbei die auftretenden Reaktionswärmen besonders günstig abgeführt werden können.

Der Umstand, daß in der ersten Reaktionsstufe des erfindungsgemäßen Verfahrens hohe Ausbeuten erzielt werden, muß als überraschend bezeichnet werden, da bei den erfindungsgemäßen Temperaturen des Verfahrens damit zu rechnen war, daß die im Verfahren benötigte Blausäure zu Ammoniumformiat hydrolisieren würde. Als überraschend muß weiterhin die Tatsache bezeichnet werden, daß bei der gleichzeitigen Dosierung der Ausgangskomponenten, CMA, Blausäure und wäßrige Schwefelsäure, bei den erfindungsgemäßen Temperaturen von 60°C bis 120°C, bevorzugt von 80°C bis 120°C, Selektivitäten größer 95 % erzielt werden. Würde die Dosierung der Ausgangskomponenten bei den erfindungsgemäßen Temperaturen in der Art und Weise, wie es in der Europäischen Patentanmeldung EP-A 0 153 561 beschrieben ist, erfolgen, wäre mit deutlich niedrigeren Selektivitäten zu rechnen, da das hierbei primär entstehende 3(4)-Aminomethyl-1-methyl-cyclohexanol (AA) durch intermolekulare Dimerisierung der gewünschten Ritterreaktion entzogen würde.

Bei dem erfindungsgemäßen Verfahren werden in der zweiten Reaktionsstufe zur Hydrolyse des in der ersten Stufe gebildeten 1-Formamido-1-methyl-3(4)-aminomethylcyclohexans und der vorhandenen überschüssigen Blausäure Wasser in der Menge zugegeben, daß sich eine Schwefelsäurekonzentration im Reaktionsgemisch von 35 bis 60 Gew.%, bevorzugt von 35 bis 45 Gew.% ergibt. Die Hydrolyse wird bei Temperaturen von 80°C bis 120°C durchgeführt.

Die zweite Reaktionsstufe des erfindungsgemäßen Verfahrens kann sowohl diskontinuierlich als auch kontinuierlich, z.B. in einer Rührkesselkaskade oder in einem Rohrreaktor, ausgeführt werden.

Die Isolierung des 1-Amino-1-methyl-3(4)-aminomethylcyclohexans (AMCA) aus dem nach der Hydrolyse erhaltenen Reaktionsgemisch erfolgt durch Extraktion. Hierzu wird das nach Hydrolyse erhaltene Reaktionsgemisch durch Basenzugabe alkalisch gestellt. Geeignete Basen sind wäßrige Ammoniaklösung und/oder Alkali- oder Erdalkalihydroxide, wie z.B. Natriumhydroxid. Wäßrige Ammoniaklösung wird in der Menge zugegeben, daß sich im Reaktionsgemisch eine Ammoniakkonzentration von 6% bis 20%, eine AMCA-Konzentration von 8 % bis 15% und eine Ammoniumsulfatkonzentration von 10 % bis 30 % ergibt. Die Menge und der Wassergehalt des zur Neutralisation verwendeten Natriumhydroxids ist dadurch bestimmt, daß das erhaltene Reaktionsgemisch einen pH-Wert von 10 bis 14, bevorzugt von 12 bis 14, eine Natriumsulfatkonzentration von 10 % bis 25 % und eine AMCA-Konzentration von 5 % bis 15% aufweist. Für die AMCA-Extraktion geeignete Extraktionsmittel sind z.B. chlorierte Kohlenwasserstoffe wie Dichlormethan oder Chlorbenzol, Kohlenwasserstoffe wie z.B. Toluol oder Xylol, Ether wie z.B. tert.-Butylmethylether, Ester wie z.B. Ethyl- oder n-Butylacetat, Ketone wie z. B. Methylisobutylketon oder Methyltertbutylketon, Alkohle wie z.B. n- und i-Butanol, 1-Pentanol, 2-Methyl-1-butanol, 2-Methyl-4-pentanol und Cyclohexanol oder Gemische derartiger Lösungsmittel, wobei Toluol, i-Butanol, Cyclohexanol und 2-Methyl-4-pentanol besonders bevorzugt sind.

Bei dem erfindungsgemäßen Verfahren kann es von Vorteil sein, die nach Hydrolyse des l-Formamido-1-methyl-3(4)-aminomethylcyclohexans (FMA) und der überschüssigen Blausäure im Reaktionsgemisch enthaltende Ameisensäure vor der AMCA-Isolierung zu entfernen. Dies kann insbesondere dann der Fall sein, wenn aus der nach der AMCA-Isolierung verbleibenden Salzlösung die Wertprodukte Ammoniumsulfat oder Natriumsulfat in reiner Form isoliert werden sollen. Die Abtrennung der Ameisensäure aus dem nach Hydrolyse erhaltenen Reaktionsgemisch erfolgt durch Extraktion wobei hierzu das Reaktionsgemisch durch Ammoniak- oder Natriumhydroxidzugabe auf einen pH-Wert von 1,3 bis 3,0 eingestellt wird. Für die Ameisensäureextraktion geeignete Extraktionsmittel sind insbesondere Alkohle wie z.B. i-Butanol, Cyclohexanol und 2-Methyl-4-pentanol.

Die nach der AMCA-Extraktion verbleibenden Salzlösungen lassen sich in geeigneter Weise zu Wertstoffen aufarbeiten. So ist es z.B. möglich, die Natriumsulfat und ggf. Natriumformiat enthaltene Salzlösung einer Elektrodialyse zu unterziehen und die hierbei ggf. nach Aufkonzentration erhaltene Schwefelsäure und Natronlauge in den Reaktionsprozeß zurückzuführen. Eine selektiv verlaufende Kristallisation von Natriumsulfat aus der Natriumsulfat und Natriumformiat enthaltenden Salzlösung ist ebenfalls möglich. Die Ammoniumsulfat und ggf. Ammoniumformiat enthaltene Salzlösung kann unter Abspaltung von zur Herstellung von Schwefelsäure wiederverwertbarem Schwefeldioxid einer thermoytischen Spaltung unterzogen werden. Durch Kristallisation ist es ebenfalls möglich, Ammoiumsulfat in reiner Form als Wertprodukt zu isolieren.

Das nach dem erfindungsgemäßen Verfahren erhaltene AMCA kann ggf. nach Entfernung des Extraktionsmittels in an sich bekannter Weise zu IMCI phosgeniert werden.

In den folgenden Beispielen beziehen sich alle Prozentangaben auf das Gewicht.

### Beispiele

### 1. Ritterreaktion

In einem Rührkessel (155ml) mit Überlauf wurden kontinuierlich 4(5)-Aminomethyl-1-methylcyclohexen (CMA), Blausäure und wäßrige Schwefelsäure gepumpt. Nach einer Versuchszeit von 60 Minuten wurden im Überlauf Selektivitäten und Umsatz mittels HPLC bestimmt. In Tab. 1 sind die Ergebnisse der einzelnen Versuche dargestellt.

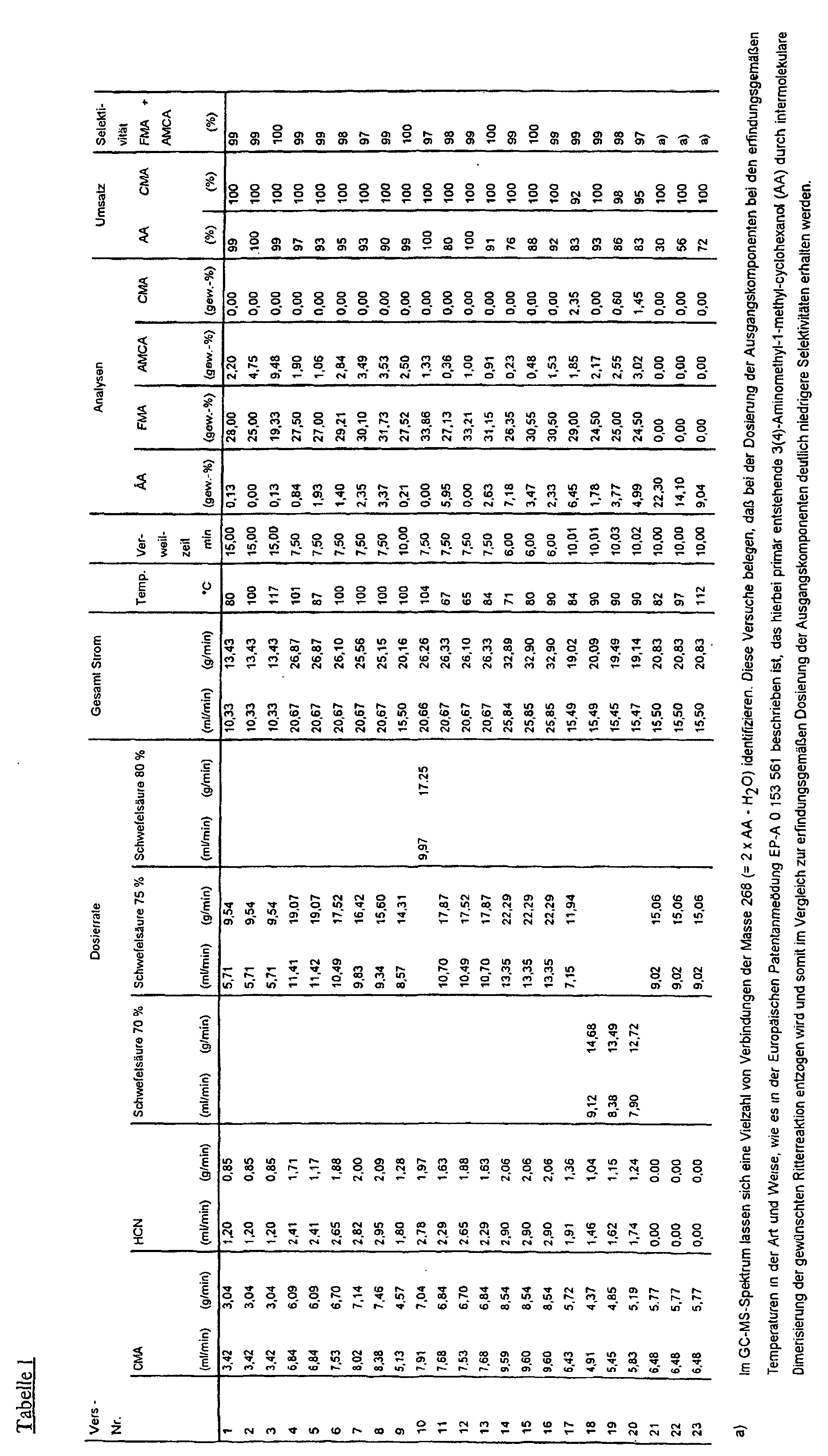

### 2. Hydrolysereaktion

Eine Mischung aus 45 % Schwefelsäure, 22,4 % Wassser, 11,7 % AMCA, 16,8 % FMA und 0,06 % Blausäure wurden 30 Minuten auf 110°C erhitzt. Die mittels HPLC durchgeführte Analyse des Reaktionsgemisches ergab einen AMCA-Gehalt von 24,84 %, FMA-Gehalt von 0,4 % und einen Blausäuregehalt von 3,8 ppm. Dies entspricht einem FMA-Umsatz von 98 % und einer AMCA-Selektivität von 96 %.

## Patentansprüche

1. Verfahren zur Herstellung von 1-Amino-1-methyl-3(4)-aminomethylcyclohexan (AMCA) **dadurch gekennzeichnet, daß** man
a) in der ersten Reaktionsstufe 4(5)-Aminomethyl-1-methylcyclohexen (CMA), Blausäure und wäßrige Schwefelsäure gleichzeitig bei Temperaturen von 60°C bis 120°C, bevorzugt bei 80°C bis 120°C umsetzt, wobei man pro Mol CMA 1-3 Mol Blansäure verwendet, und
b) in der zweiten Reaktionsstufe nach vorheriger Wasserzugabe das in der ersten Reaktionsstufe gebildete 1-Formamido-1-methyl-3(4)-aminomethylcyclohexan (FMA) und nicht umgesetzte Blausäure hydrolisiert und
c) aus dem in der zweiten Reaktionsstufe erhaltenen Reaktionsgemisch 1-Amino-1-methyl-3(4)-aminomethylcyclohexan (AMCA) nach vorhergehender Basenzugabe und ggf. nach vorheriger Ameisensäureentfernung durch Extraktion isoliert.

2. Verfahren gemäß Anspruch 1, **dadurch gekennzeichnet, daß** man CMA, Blausäure und Schwefelsäure im Molverhältnis CMA/HCN/H₂SO₄ von 1/1,1-1,7/2,2-2,7 umsetzt und den Wassergehalt der verwendeten Schwefelsäure so einstellt, daß sich ein Molverhältnis CMA/H₂O von ½ bis 1/7, bevorzugt von 1/3 bis 1/6 ergibt.

3. Verfahren gemäß Anspruch 1, **dadurch gekennzeichnet, daß** die Wasserzugabe in der zweiten Reaktionsstufe in der Menge erfolgt, daß sich im Reaktionsgemisch eine Schwefelsäurekonzentration von 35 bis 60 Gew.%, bevorzugt von 35 bis 45 Gew.% ergibt.

4. Verfahren gemäß Anspruch 1, **dadurch gekennzeichnet, daß** man zur Neutralisation des nach Hydrolyse erhaltenen Reaktionsgemisches wäßrige Ammoniaklösung in der Menge verwendet, daß sich im erhaltenen Reaktionsgemisch eine Ammoniakkonzentration von 6 % bis 20 %, eine AMCA-Konzentration von 8 % bis 15 % und eine Ammoniumsulfatkonzentration von 10 % bis 30 % ergibt.

5. Verfahren gemäß Anspruch 1, **dadurch gekennzeichnet, daß** man zur AMCA-Extraktion 2-Methyl-4-pentanol als Extraktionsmittel verwendet.

6. Verfahren gemäß Anspruch 1, **dadurch gekennzeichnet, daß** sämtliche Verfahrensschritte kontinuierlich ausgeführt werden.

## Claims

1. Process for the production of 1-amino-1-methyl-3(4)-aminomethylcyclohexane (AMCA), **characterised in that**
a) in the first reaction stage 4(5)-aminomethyl-1-methylcyclohexene (CMA), hydrocyanic acid and aqueous sulfuric acid are caused to react simultaneously at temperatures from 60°C to 120°C, preferably at 80°C to 120°C, use being made of 1-3 moles of hydrocyanic acid per mole of CMA, and
b) in the second reaction stage, after previous addition of water the 1-formamido-1-methyl-3(4)-aminomethylcyclohexane (FMA) that is formed in the first reaction stage and non-reacted hydrocyanic acid are hydrolysed and
c) 1-amino-1-methyl-3(4)-aminomethylcyclohexane (AMCA) is isolated by extraction from the reaction mixture obtained in the second reaction stage after prior addition of a base and optionally after previous removal of formic acid.

2. Process according to Claim 1, **characterised in that** CMA, hydrocyanic acid and sulfuric acid are caused to react in a molar ratio CMA/HCN/H₂SO₄ of 1/1.1-1.7/2.2-2.7 and the water'content of the sulfuric acid that is used is adjusted so that a molar ratio CMA/H₂O from % to 1/7, preferably from 1/3 to 1/6, arises.

3. Process according to Claim 1, **characterised in that** the addition of water in the second reaction stage is effected in such a quantity that in the reaction mixture a concentration of sulfuric acid from 35 to 60 wt.%, preferably from 35 to 45 wt.%, arises.

4. Process according to Claim 1, **characterised in that** for the purpose of neutralisation of the reaction mixture obtained after hydrolysis use is made of aqueous ammonia solution in such a quantity that an ammonia concentration from 6 % to 20 %, an AMCA concentration from 8 % to 15 % and an ammonium-sulfate concentration from 10 % to 30 % arise in the reaction mixture that is obtained.

5. Process according to Claim 1, **characterised in that** for the purpose of extraction of AMCA use is made of 2-methyl-4-pentanol as extracting agent.

6. Process according to Claim 1, **characterised in that** all the process steps are carried out continuously.

## Revendications

1. Procédé pour la préparation du 1-amino-1-méthyl-3(4)-aminométhylcyclohexane (AMCA), **caractérisé en ce que**
a) dans la première étape réactionnelle, on fait réagir du 4(5)-aminométhyl-1-méthylcyclohexène (CMA), de l'acide cyanhydrique et de l'acide sulfurique aqueux de manière simultanée à des températures de 60 °C à 120 °C, de préférence de 80 °C à 120 °C, en utilisant, par mole de CMA, de 1 à 3 moles d'acide cyanhydrique, et
b) dans la deuxième étape réactionnelle, après addition préalable d'eau, on soumet à une hydrolyse le 1-formamido-1-méthyl-3(4)-amino-méthylcyclohexane (FMA) obtenu dans la première étape réactionnelle et l'acide cyanhydrique n'ayant pas réagi, et
c) à partir du mélange réactionnel obtenu dans la deuxième étape réactionnelle, on isole par extraction le 1-amino-1-méthyl-3(4)-amino-méthylcyclohexane (AMCA), après addition préalable d'une base et le cas échéant, après élimination préalable de l'acide formique.

2. Procédé selon la revendication 1, **caractérisé en ce qu'**on fait réagir du CMA, de l'acide cyanhydrique et de l'acide sulfurique dans le rapport molaire CMA/HCN/H₂SO₄ de 1/1,1 - 1,7/2,2 - 2,7 et on règle la teneur en eau de l'acide sulfurique utilisé de telle sorte que l'on obtient un rapport molaire CMA/H₂O de 1/2 à 1/7, de préférence de 1/3 à 1/6.

3. Procédé selon la revendication 1, **caractérisé en ce que** l'addition d'eau dans la deuxième étape réactionnelle a lieu en une quantité telle que l'on obtient, dans le mélange réactionnel, une concentration d'acide sulfurique de 35 à 60 % en poids, de préférence de 35 à 45 % en poids.

4. Procédé selon la revendication 1, **caractérisé en ce qu'**on utilise, à des fins de neutralisation du mélange réactionnel obtenu après l'hydrolyse, une solution aqueuse d'ammoniaque telle que l'on obtient dans le mélange réactionnel obtenu, une concentration d'ammoniaque de 6 % à 20 %, une concentration de AMCA de 8 % à 15 % et une concentration de sulfate d'ammonium de 10 % à 30 %.

5. Procédé selon la revendication 1, **caractérisé en ce qu'**on utilise pour l'extraction du AMCA, du 2-méthyl-4-pentanol, à titre d'agent d'extraction.

6. Procédé selon la revendication 1, **caractérisé en ce qu'**on met en oeuvre toutes les étapes opératoires en continu.
